# EUROPÄISCHE PATENTSCHRIFT

(11) **EP 2 636 376 B1**
(45) Veröffentlichungstag und Bekanntmachung des Hinweises auf die Patenterteilung: **21.01.2015**
(21) Anmeldenummer: 13158180.3
(22) Anmeldetag: 07.03.2013
(51) Int. Cl.: A61B 17/06

(54) **Nadel-Lift**
Needle lift
Emballage pour aiguille avec un moyen de soulèvement

(30) Priorität: 09.03.2012 DE 102012004558
(43) Veröffentlichungstag der Anmeldung: 11.09.2013
(73) Patentinhaber: AESCULAP AG, 78532 Tuttlingen (DE)
(72) Erfinder: Koller, Tobias, 78532 Tuttlingen (DE)

(56) Entgegenhaltungen:
- EP-A1- 0 060 080
- WO-A1-01/24710
- DE-A1- 2 902 048

## Beschreibung

Die vorliegende Erfindung betrifft einen Nadel-Lift für chirurgisches Nahtmaterial welcher das Greifen und Entnehmen der Nadel und des chirurgischen Nahtmaterials aus der Nahtmaterialverpackung erleichtert, sowie eine Nahtmaterialverpackung mit einem Nadel-Lift.

### Stand der Technik

Chirurgisches Nahtmaterial wird zurzeit in sogenannten RacePacks vertrieben. Diese RacePacks sind im Wesentlichen ovale Verpackungen aus Kunststoff, auf welche das Nahtmaterial automatisch aufgewickelt wird und an deren Oberfläche sich eine Nadelhalterung befindet. Meistens ist das Nahtmaterial in einem ebenfalls ovalen und entlang des Randes der Verpackung verlaufenden Kanal aufgenommen, der von einem Bodenbauteil und einem Deckelbauteil gebildet wird. Die Nadel ist zumeist mit Hilfe einer Dreipunktlagerung gesichert, in welche die Nadel des chirurgischen Nahtmaterials eingesteckt wird. Eine solche Verpackung ermöglicht ein vollautomatisches Wickeln des Nahtfadens sowie ein vollautomatisches Einbringen der Nadel in die Nadelhalterung.

Die Verpackung und das Nahtmaterial zusammen werden steril in einer Umverpackung beispielsweise aus Aluminiumfolie verpackt. Bei der Verwendung eines solchen Nahtmaterials während einer Operation öffnet eine unsterile Schwester die Umverpackung etwa zur Hälfte und bietet dem Operateur oder einer sterilen Schwester die Nahtmaterialverpackung mit dem Nahtmaterial an. Der Operateur oder die sterile Schwester legt die aus der Verpackung entnommene Nahtmaterialverpackung meistens zusammen mit weiteren Nahtmaterialverpackungen ab. Wird ein Nahtmaterial letztlich benötigt, reicht die sterile Schwester dem Operateur die Nahtmaterialverpackung und der Operateur greift dann mit einem Nadelhalter nach der Nadel und zieht die Nadel mitsamt Faden aus der Verpackung. Manche Nahtmaterialverpackungen verfügen in dem Bereich, in dem das Greifen der Nadel beabsichtigt ist, über ein Durchgangsloch oder eine Klappe, welche nach hinten ausweichen kann, um dem Operateur das Greifen der Nadel zu vereinfachen. Ohne Loch oder Klappe besteht nur ein sehr kleiner Abstand zwischen der Nadel und der Grundfläche der Verpackung. Dies bedeutet, dass der Operateur die Nadel mit seinem Nadelhalter nur an der äußersten Spitze des Nadelhalters greifen kann. Hierzu muss der Operateur sehr präzise vorgehen und die Nadel kann auch sehr leicht aus dem Maul des Nadelhalters herausrutschen, wenn der Operateur auch nur ganz kurz die erforderliche Haltekraft nicht aufbringt. Nadelhalter verfügen gewöhnlich über Vorrichtungen, die ein Öffnen des Maulteils während des Einsatzes verhindern. Aber je nach Art des Nadelhalters muss dazu eine Kraft bzw. sogar eine Verformung auf den Nadelhalter aufgebracht werden, was direkt während der Entnahme der Nadel aus der Nahtmaterialverpackung meist noch nicht geschieht.

Verfügt die Verpackung über ein Loch oder eine ausweichende Klappe, kann der Operateur die Nadel mit einem mittiger gelegenen Abschnitt des Maulteils des Nadelhalters greifen, sodass obige Probleme nicht in diesem Maße auftreten. Allerdings bergen diese Maßnahmen auch Nachteile. Der offensichtlichste Nachteil ist, dass die Öffnung des Maulteils während des Greifvorgangs sehr beschränkt ist, da diese von der Größe des Lochs bzw. der Klappe abhängt. Der Operateur stößt also häufig gegen die Grundfläche der Verpackung. Insbesondere wenn eine Klappe vorgesehen ist, muss der Operateur diese zunächst nach hinten wegbiegen, was ebenfalls eine gewisse Kraft erfordert. Der Operateur muss also eine gewisse Kraft in der Instrumentenführung (d.h. in der Führung des Nadelhalters) einsetzen. Stößt der Operateur aber mit dem Nadelhalter zu stark gegen die Nadel, kann sich diese aus der Nadelhalterung lösen. Dann baumelt sie lose am Faden und ist sehr schwer zu greifen. Außerdem stellt die Nadel dann eine gewisse Verletzungsgefahr dar.

Aus diesem Grund sind einige Operationsteams dazu übergegangen, die Verpackungen mit einer Klappe anders zu nutzen. Wenn die sterile Schwester dem Operateur die Nahtmaterialverpackung gibt oder hinhält, drückt sie zuvor mit einem Finger von hinten gegen die Klappe. Auf diese Weise wird die Nadel ein wenig angehoben und der abstand zwischen Nadel und Grundfläche vergrößert sich zumindest ein wenig. Diese Handhabung hat aber auch zahlreiche Nachteile. Zum einen kann, da die Nadelhalterung an der Grundfläche vorgesehen ist und nicht an der Klappe, die Nadel aus der Nadelhalterung herausrutschen, wenn die Klappe zu weit nach vorn gedrückt wird. Zum anderen sind für das vordrücken der Klappe filigrane und feinfühlige Finger erforderlich, da die Klappen meist recht klein ausgebildet sind. Nachteilig ist aber insbesondere auch, dass sich die Klappe immer unterschiedlich verhält. Mal bleibt sie relativ weit vorgebogen stehen, mal kehrt sie fast vollständig in ihre Ursprungsposition zurück. Die Nadel nimmt auf diese Weise also keine definierte Position ein, sodass kaum eine Routine beim Entnehmen der Nadel auf diese Weise entstehen kann. DE 290248 offenbart einen Nadelhalter entsprechend dem Obergriff von Anspruch 1.

Die Aufgabe der vorliegenden Erfindung ist es daher, eine Möglichkeit zu schaffen, den zu greifenden Bereich der Nadel einfach, schnell, sicher und kraftarm in einer definierten Stellung über der Grundfläche zu greifen, sodass Fehler beim Greifen der Nadel vermieden werden können und so wertvolle Zeit eingespart werden kann.

Die Aufgabe der vorliegenden Erfindung wird gelöst durch einen Nadel-Lift nach einem der Ansprüche 1 und 4 sowie eine Nahtmaterialverpackung nach Anspruch 11. Weitere vorteilhafte Fortbildungen sind Gegenstand der abhängigen Ansprüche.

Gemäß einem ersten Aspekt der vorliegenden Erfindung ist ein Nadel-Lift für chirurgisches Nahtmaterial offenbart mit einer Grundfläche und einem bewegbaren Bauteil, das zumindest teilweise gegenüber einer Grundfläche in der Ebene der Grundfläche bewegbar ist, wobei das bewegbare Bauteil so geformt ist und so an der Grundfläche befestigt ist, dass eine Bewegung des bewegbaren Bauteils in der Ebene der Grundfläche dazu führt, dass sich zumindest ein Teil des bewegbaren Bauteils von der Grundfläche abhebt. Dabei ist eine Nadelhalterung in dem Bereich des bewegbaren Bauteils vorgesehen, der sich von der Grundfläche abhebt. Darüber hinaus weist das Bewegbare Bauteil mindestens eine Zuglasche mit einem proximalen Ende, einem Mittelabschnitt und einem distalen Ende auf, wobei die mindestens eine Zuglasche im Bereich ihres distalen Endes an einer Grundfläche verschiebbar gehalten ist. Der Nadel-Lift weist zudem mindestens einen Knickabschnitt auf, der mit seinem distalen Ende an dem Mittelabschnitt der mindestens einen Zuglasche befestigt ist und mit seinem proximalen Ende an einer Grundfläche befestigt ist. Eine Nadelhalterung für eine chirurgische Nadel ist zudem an dem mindestens einen Knickabschnitt vorgesehen.

Gemäß einer solchen Vorrichtung, welche die Nadel von der Grundfläche, auf der sich die Nadel normalerweise befindet, weg bewegt, wird eine einfache, leichte, schnelle, sichere und kostengünstige Möglichkeit für den Operateur geboten, die Nadel mit einem Nadelhalter aufzunehmen. Dieser Aspekt umfasst verschiedenste Möglichkeiten, wie ein Nadel-Lift ausgeführt werden kann. Das grundlegende Prinzip besteht darin, dass eine Bewegung innerhalb einer Ebene parallel zu der Grundfläche zumindest teilweise in eine Bewegung umgewandelt wird, die senkrecht zu der Ebene der Grundfläche verläuft. Eine Nahtmaterialverpackung ist ein sehr dünnes und flächiges Objekt, welches zumeist eine im Wesentlichen ovale und sehr dünne Form hat. Gewöhnlich besteht eine Nahtmaterialverpackung aus sehr wenigen Einzelteilen, üblicherweise nur einem Bodenbauteil und einem Deckelbauteil, die miteinander verbunden sind, in ihrem Randbereich einen Fadenkanal bilden und bei denen im Innenbereich des Fadenkanals eine oder mehrere Nadelhalterungen angeordnet sind.

Um eine Bewegung aus einer Ebene heraus durch eine Bewegung innerhalb einer Ebene zu erzeugen wird das Prinzip der Stauchung bzw. Abstandsverkürzung verwendet. Der Abstand zweier Punkte, die in einer Ebene liegen, wird verkürzt. Da die beiden Punkte durch ein Element verbunden sind, welches sich nicht in ausreichendem Maße verkürzen kann, weicht das Element in seinem zentralen Bereich seitlich aus. Damit das Element nicht innerhalb der Ebene seitlich ausweicht, sondern aus der Ebene heraus sind die Biegesteifigkeiten innerhalb der Ebene höher zu wählen bzw. einzustellen als senkrecht dazu. Dies wird leicht erreicht, indem das Element eine größere Breite in der Ebene als Dicke senkrecht dazu aufweist. Auf diese Weise ist die Biegesteifigkeit des Elements innerhalb der Ebene größer als senkrecht dazu und es wird aus der Ebene heraus ausweichen. Vorstehendes gilt für alle isotropen Werkstoffe des Elements. Im Prinzip kann aber auch ein anisotroper Werkstoff verwendet werden, der einen unterschiedlichen Elastizitätsmodul in verschiedene Raumrichtungen aufweist, wobei sich dann die Abmessungen der Breite und der Dicke nicht unbedingt unterscheiden müssen und sogar die Dicke größer als die Breite sein kann. Maßgeblich ist letztendlich ausschließlich das maximal aufnehmbare Biegemoment (M), welches sich aus dem Elastizitätsmodul (E) und den Querschnittsabmessungen (h und b) des Elements ergibt (M=E*h³*b/12).

Wenn ein solches Element gestaucht wird, d.h. der Abstand der beiden Endpunkte zueinander verringert wird, kann der mittlere Bereich auf zwei verschiedene Arten ausweichen. Ist die Biegesteifigkeit entlang des Elements im Wesentlichen gleichbleibend und die Biegebelastung des Elements nicht zu groß, so biegt sich das Element in Abhängigkeit von den Randbedingungen im Wesentlichen gleichmäßig durch. Das Element weicht also durch Biegen aus.

Es gibt zudem zahlreiche Möglichkeiten, ein Element zu stauchen, d.h. den Abstand seiner Endpunkte zueinander zu verringern. Die einfachste Möglichkeit besteht darin, die beiden Endpunkte zueinander hin zu schieben. Eine weitere Möglichkeit besteht darin, an beiden Enden des Elements ein Zugelement anzubringen, welches zu dem jeweils gegenüberliegenden Ende des Elements verläuft und dann an den beiden freien Enden des Zugelements zu ziehen. Selbstverständlich kann in beiden Fällen ein Ende des Elements fixiert sein, sodass nur an einem Ende des Elements eine Verschiebung auftritt.

Bei einer solchen Ausführungsform wird, wenn an der Zuglasche gezogen wird, die Nadelhalterung mit ggf. einer chirurgischen Nadel in der Zugrichtung bewegt. Gleichzeitig versucht der Knickabschnitt seitlich auszuweichen, da sich seine beiden Endabschnitte in der Zugrichtung aneinander annähern. Da der Knickabschnitt eine beachtliche Breite aber nur eine sehr geringe Dicke aufweist, wird der Knickabschnitt versuchen, senkrecht zur Grundfläche auszuweichen. Nach unten kann er aber nicht ausweichen, da sich dort die Grundfläche befindet, an der der Knickabschnitt anliegt. Die Grundfläche ist hierbei als ausreichen steif zu betrachten, sodass diese nicht durch den Knickabschnitt verformt wird. Letztlich bleibt dem Knickabschnitt nur die Möglichkeit, nach oben auszuweichen. Auf diese Weise wird die Nadelhalterung und ggf. die darin befindliche Nadel von der Grundfläche abgehoben.

Gemäß einer vorteilhaften Ausführungsform des ersten Aspekts der vorliegenden Erfindung verfügt der mindestens eine Knickabschnitt über wenigstens eine Soll-Knickstelle, die im Wesentlichen quer zur Längsachse der mindestens einen Zuglasche verläuft.

Verfügt das Element über eine Art Gelenk, also über eine oder mehrere Stellen, an denen die Biegesteifigkeit wesentlich geringer als in deren Umgebung ist, so wird das Element an dieser Stelle knicken, sich also an dieser Stelle ungleichmäßig biegen. Abhängig vom verwendeten Material wird die Biegung am Knick eine im Wesentlichen plastische Verformung sein. Abhängig von der Lagerung der Endabschnitte des Elements, also den Randbedingungen, werden eine, zwei oder gar drei solcher Soll-Knickstellen benötigt, um ein Ausweichen nur aufgrund von Knicken zu ermöglichen. Es ist aber auch möglich, die beiden Ausweicharten zu kombinieren, indem z.B. bei einer biegefesten Lagerung der Endabschnitte des Elements zwei Soll-Knickstellen vorgesehen werden und ein Biegebereich, in dem sich das Element gleichmäßig biegt. Der Bereich zwischen den beiden Soll-Knickstellen stellt dabei eine Pendelstütze dar und ist im Wesentlichen momentenfrei und daher nicht gebogen.

Gemäß einer besonderen Ausführungsform des ersten Aspekts der vorliegenden Erfindung verfügt der mindestens eine Knickabschnitt über drei Soll-Knickstellen und die Nadelhalterung besteht aus einer Dreipunktlagerung, welche die Nadel so lagert, dass sie die mittlere Soll-Knickstelle quert.

Bei einer solchen Ausführungsform mit drei Soll-Knickstellen ist der Knickabschnitt auch nach der Bewegung und Verformung weitestgehend momentenfrei und kraftfrei, da die drei Soll-Knickstellen zwei Pendelstützen definieren, welche quasi gelenkig verbunden sind. Eine Dreipunktlagerung ist als Nadelhalterung besonders geeignet, da in dieser die Kraftverhältnisse und die Position der Nadel statisch eindeutig bestimmt sind. Die Dreipunktlagerung als Nadelhalterung kann für alle Nadel-Lifte verwendet werden und ist besonders vorteilhaft, es können aber auch bei allen Nadel-Lifte überbestimmende Nadelhalterungen eingesetzt werden, so z.B. Halterungen mit vier oder mehr Kontaktpunkten oder weitere im Stand der Technik bekannte Nadelhalterungen.

Gemäß einem zweiten Aspekt der vorliegenden Erfindung ist ein Nadel-Lift für chirurgisches Nahtmaterial vorgesehen mit einer Grundfläche und mindestens einer Zuglasche mit einem proximalen Ende, einem Mittelabschnitt und einem distalen Ende, wobei die mindestens eine Zuglasche im Bereich ihres distalen Endes an einer Grundfläche befestigt ist und die mindestens eine Zuglasche im Bereich ihres proximalen Endes an einer Grundfläche verschiebbar gehalten ist. Zudem ist der Mittelabschnitt als mindestens ein "S"-förmiger Abschnitt ausgebildet, welcher das proximale und das distale Ende der Zuglasche verbindet. An dem mindestens einen "S"-förmigen Abschnitt ist eine Nadelhalterung für eine chirurgische Nadel vorgesehen. Eine Nadelhalterung für eine chirurgische Nadel ist zudem an dem mindestens einen Knickabschnitt vorgesehen.

Bei dieser Ausführungsform besteht ein "S"-förmiger Abschnitt aus einem Bereich, der sich vom distalen Ende zu dem proximalen Ende hin erstreckt, dann eine Kurve macht bzw. Biegung (nicht im Sinne von Verbiegung) hat, sich von dort aus zurück zu dem distalen Ende hin erstreckt, dann wieder eine Kurve macht bzw. eine Biegung hat, und sich danach wiederum zu dem proximalen Ende hin erstreckt. Auf diese Weise verfügt solch ein "S"-förmiger Abschnitt über ein distales Ende und ein proximales Ende, aber auch über eine distale Biegung und eine proximale Biegung. Bei dieser Ausführungsform wird die distale Biegung zu der proximalen Biegung gezogen und so ein Ausweichen der Nadelhalterung aus der Ebene der Grundfläche provoziert. Wenn die distale und die proximale Biegung frei sind, d.h. senkrecht zu der Ebene der Grundfläche nicht gehalten sind, wird entweder die distale oder die proximale Biegung ausweichen. Daher wird die distale Biegung in der Nähe der Grundfläche gehalten, indem beispielsweise eine kragarmförmige Lasche in der Grundfläche ausgebildet wird und entweder das distale Ende der Zuglasche seitlich zwischen Grundfläche und freiem Ende der Lasche positioniert wird, oder zum Beispiel die distale Biegung von distaler Richtung her von einer solchen Lasche in der Nähe der Grundfläche gehalten wird. Anstelle der in der Grundfläche ausgebildeten Laschen sind auch Brückenbauteile verwendbar, wie dies bei der zweiten Ausführungsform beschrieben ist. Solche Brückenbauteile können einstückig mit der Zuglasche ausgebildet sein, können aber auch separate Bauteile sein oder durch einen Teil des Bodenbauteils oder des Deckelbauteils gebildet werden, wie dies später genauer erläutert ist.

Bei an der Grundfläche gehaltener distaler Biegung weicht nun, wenn die Zuglasche gezogen wird, die proximale Biegung nach oben aus und ermöglicht einen komfortablen Zugriff auf die Nadel.

Diese Ausführungsform verwendet vorzugsweise ausschließlich elastische Verformungen, d.h. keine Soll-Knickstellen. Es können jedoch auch Soll-Knickstellen verwendet werden, um eine kraftärmere Betätigung zu ermöglichen. Solche Soll-Knickstellen verlaufen dann im Wesentlichen quer zur Längsachse der Zuglasche, welche mit der Zugrichtung übereinstimmt. Diese Soll-Knickstellen sind vorzugsweise im Bereich der distalen Biegung und/oder der proximalen Biegung vorgesehen, bevorzugt verlaufen sie durch den Innenrand der jeweiligen Biegung (quasi dem Endpunkt des Schlitzes, der die jeweiligen Schenkel der Biegung voneinander trennt), entweder nur auf einer Seite der Biegung oder aber auf beiden Seiten der Biegung. Auch im Bereich der distalen Befestigung der Zuglasche kann eine Soll-Knickstelle vorgesehen sein.

Gemäß einer weiteren Ausführungsform des ersten und zweiten Aspekts der vorliegenden Erfindung ist die Zuglasche an der Grundfläche verschiebbar gehalten, indem in der Grundfläche kragarmförmige Laschen ausgebildet sind, welche beiderseits der Zuglasche angeordnet sind, wobei die Zuglasche zwischen den freien Enden der kragarmförmigen Laschen und der Grundfläche angeordnet ist.

Gemäß einer besonderen Ausführungsform des zweiten Aspekts der vorliegenden Erfindung besteht die Nadelhalterung aus einer Dreipunktlagerung, welche die Nadel so lagert, dass sich das der Nadelspitze entgegengesetzte Ende der Nadel außerhalb des "S"-förmigen Bereichs zu dem proximalen Ende der mindestens einen Zuglasche hin befindet. Auf diese Weise kann die nadel vom Operateur besonders einfach gegriffen werden, da sie nach einer Betätigung des Nadel-Lifts weit über der Grundfläche positioniert ist. Alternativ dazu kann der "S"-förmige Bereich so von seinem proximalen Ende her geschlitzt sein, dass ein Nadelhalter in diesen Schlitz eingeführt werden kann, um die Nadel dort zu greifen. Bei der Verwendung mehrerer "S"-förmiger Bereiche kann ein solcher Schlitz auch zwischen den "S"-förmigen Bereichen ausgebildet sein.

Gemäß einer besonderen Ausführungsform des ersten und zweiten Aspekts der vorliegenden Erfindung verfügt die mindestens eine Zuglasche über ein Griffelement.

Wenn ein solches Griffelement vorgesehen ist, erleichtert dies das Betätigen der Zuglasche. Insbesondere erleichtert ein Griffelement die gleichzeitige Betätigung mehrerer Zuglaschen, falls mehrere Zuglaschen vorgesehen sind.

Gemäß einer besonderen Ausführungsform des ersten und zweiten Aspekts der vorliegenden Erfindung verfügt die mindestens eine Zuglasche und/oder die Grundfläche über eine Einrichtung, welche die Zuglasche nach einer Verschiebung derselben in der verschobenen Stellung hält.

Dies kann beispielsweise ein Vorsprung sein, der aus der Grundfläche hervorsteht und in der Ausgangsstellung leicht gegen die Unterseite des Nadel-Lifts drückt. Wird der Nadel-Lift nun betätigt, wird dieser Vorsprung freigelegt. Will der nadel-Lift nun in seine Ausgangsstellung zurück, stößt eine distale Kante der Zuglasche gegen diesen Vorsprung und verhindert, dass der Nadel-Lift weiter in seine Ausgangsposition zurück kehrt. Besonders vorteilhaft ist solch ein Vorsprung bei der dritten Ausführungsform im Bereich der distalen Biegung. Diese wird aufgrund der elastischen Verformung relativ stark gegen die Grundfläche gedrückt, sodass der Vorsprung relativ klein sein kann und trotzdem ein Rückkehren des Nadel-Lifts in die Ausgangsposition sicher verhindern kann. Eine andere Art einer solchen Einrichtung kann ein Reibeelement sein, welches eine höhere Haftkraft bzw. Reibkraft als die Rückstellkraft des Nadel-Lifts aufweist. Ein solches Reibelement kann auch durch eines der Brückenbauteile ausgebildet sein, wie sie bei der zweiten Ausführungsform beschrieben wurden, aber auch durch seitliche Vorsprünge an der Zuglasche, die in oder an passenden Gegenstücken einrasten, welche z.B. an der Grundfläche vorgesehen oder direkt oder indirekt befestigt sind. Prinzipiell kann eine solche Einrichtung entlang der gesamten Zuglasche angeordnet sein, besonders vorteilhaft ist sie allerdings in der Nähe des Bereichs angeordnet, der sich von der Grundfläche abhebt, da dort auch die Rückstellkräfte in der Zuglasche gespeichert sind.

Gemäß einer besonderen Ausführungsform des ersten und zweiten Aspekts der vorliegenden Erfindung ist die mindestens eine Zuglasche an der Grundfläche verschiebbar gehalten, indem ein Brückenbauteil über die mindestens eine Zuglasche verläuft, welches beiderseits der mindestens einen Zuglasche an der Grundfläche befestigt ist, wobei das Brückenbauteil vorzugsweise ein Teil der mindestens einen Zuglasche selbst ist, welches umgefaltet, umgebogen bzw. umgeknickt ist, breiter als der angrenzende Bereich der mindestens einen Zuglasche ist, und auf dem einem Teil der mindestens einen Zuglasche liegt.

Bei einer solchen Ausführungsform dient das distale Ende der mindestens einen Zuglasche dazu, den Bereich der Zuglasche, an dem der Knickabschnitt angebracht ist, und somit das distale Ende des Knickabschnitts selbst in der Nähe der Grundfläche zu halten. Dies ist erforderlich, um sicher zu stellen, dass nicht der distale Bereich des Knickabschnitts ausweicht, sondern der mittlere Abschnitt des Knickabschnitts. Wenn das distale Ende des Knickabschnitts, welches nicht wie das proximale Ende des Knickabschnitts an der Grundfläche fixiert ist, nicht in der Nähe der Grundfläche gehalten wird, kann es sein, dass sich das distale Ende des Knickabschnitts von der Grundfläche abhebt, was zur Folge hat, dass sich die Nadelhalterung nicht mehr unbedingt von der Grundfläche abhebt. Dies ist unbedingt zu verhindern. Im vorliegenden Fall wird dies verhindert, indem das distale Ende der Zuglasche und somit auch das distale Ende des Knickabschnitts mit Hilfe einer Art Brückenbauteil an der Grundfläche befestigt wird. Das distale Ende der Zuglasche weist eine Verbreiterung auf. Diese Verbreiterung wird zu dem proximalen Ende hin umgefaltet, umgebogen bzw. umgeknickt, wobei ein Teil der mindestens einen Zuglasche eine Art Schlaufe bildet. Diese Schlaufe stellt sicher, dass die mindestens eine Zuglasche noch ausreichend innerhalb einer Ebene parallel zu der Grundfläche in Richtung zu dem proximalen Ende der mindestens einen Zuglasche hin bewegbar bleibt. An den lateralen Bereichen der Verbreiterung ist das distale Ende der mindestens einen Zuglasche an der Grundfläche fixiert.

Gemäß einer anderen besonderen Ausführungsform des ersten und zweiten Aspekts der vorliegenden Erfindung ist die Zuglasche an der Grundfläche verschiebbar gehalten, indem in der mindestens einen Zuglasche ein Schlitz entlang ihrer Längsachse vorgesehen ist und in der Grundfläche kragarmförmige Laschen ausgebildet sind, welche sich in beide Richtungen quer zur Längsachse der mindestens einen Zuglasche durch den Schlitz erstrecken. Dabei ist die mindestens eine Zuglasche zwischen den freien Enden der kragarmförmigen Laschen und der Grundfläche angeordnet.

Auf diese Weise kann die Zuglasche in der Nähe der Grundfläche gehalten werden, ohne dass weitere Bauteile wie Brückenelemente vorgesehen werden müssen oder die Zuglasche vor einer Montage erst gefaltet, gebogen oder geknickt werden muss. Die kragarmförmigen Laschen können in die Grundfläche gestanzt werden und für eine Montage des Nadel-Lifts mit Hilfe von Stiften von unten elastisch nach oben gebogen werden. Anschließend wird der Nadellift auf die Grundfläche aufgesetzt, an den vorgesehenen Stellen an der Grundfläche fixiert und die kragarmförmigen Laschen frei gegeben. Dann ist die mindestens eine Zuglasche mit Hilfe der Laschen verschiebbar gelagert und gegen Abheben gesichert.

Gemäß einem dritten Aspekt der vorliegenden Erfindung ist eine Nahtmaterialverpackung vorgesehen mit einem Bodenbauteil und einem Deckelbauteil, wobei zwischen Bodenbauteil und Deckelbauteil zumindest teilweise ein Aufnahmeraum für den Faden ausgebildet ist. Eine Grundfläche, welche an dem Bodenbauteil oder dem Deckelbauteil vorgesehen ist, und ein Nadel-Lift nach einem der vorstehenden Aspekte sind ebenfalls vorgesehen.

Mit einer solchen Nahtmaterialverpackung inklusive Nadel-Lift kann ein Operateur ein Nahtmaterial besonders leicht aus der Nahtmaterialverpackung entnehmen, da es nicht lange und kompliziert nach einem guten Griff für die Nadel suchen muss. Die nadel liegt zum großen Teil frei und deutlich vor der Grundfläche. Es spielt in den meisten Ausführungsformen überhaupt keine Rolle, wie weit der Nadelhalter geöffnet ist, in einigen wenigen Ausführungsformen ist die maximale Öffnung des Nadelhalters zwar begrenzt, aber deutlich weiter als bei den bisherigen Nahtmaterialverpackungen. Außerdem muss der Operateur beim entnehmen der Nadel keine Klappe wegdrücken, sodass er die Nadel quasi kraftfrei aufnehmen kann. Daher wird er sofort bemerken, wenn er versehentlich gegen die Nadel stößt, sodass nicht mehr die Gefahr besteht, dass er versehentlich mit dem Nadelhalter die Nadel aus der Nadelhalterung drückt.

Gemäß einer besonderen Ausführungsform des dritten Aspekts der vorliegenden Erfindung ist die Grundfläche an dem Bodenbauteil vorgesehen und der Nadel-Lift befindet sich zwischen Bodenbauteil und Deckelbauteil, sodass die Bereiche der Zuglasche, welche gegenüber der Grundplatte verschiebbar gehalten sind, durch das Deckelbauteil gehalten sind.

Auf diese Weise werden besonders wenige Teile benötigt. Genau genommen kann auf diese Weise eine komplette Nahtmaterialverpackung mit Nadel-Lift hergestellt werden, die nur ein einziges Bauteil mehr als eine herkömmliche Nahtmaterialverpackung aufweist. Dies sorgt dafür, dass eine solche Nahtmaterialverpackung einfach und kostengünstig produziert werden kann.

Gemäß einer besonderen Ausführungsform des dritten Aspekts der vorliegenden Erfindung ist ein Beschriftungsbauteil auf dem Deckelbauteil vorgesehen, welches zumindest die Nadelspitze auch dann noch abdeckt, wenn der Nadel-Lift betätigt wurde.

Auf diese Weise kann eine Verletzungsgefahr durch die Nadel besonders gut verhindert werden, bis der Operateur die Nadel aufgenommen hat.

Weitere Vorteile und Merkmale der Erfindung sind dem Fachmann aus den beigefügten Figuren und der detaillierten Beschreibung der Ausführungsbeispiele ersichtlich.
- Fig. 1A: zeigt ein erstes Ausführungsbeispiel der vorliegenden Erfindung im unbetätigten Zustand von oben;
- Fig. 1B: zeigt ein erstes Ausführungsbeispiel der vorliegenden Erfindung im unbetätigten Zustand von der Seite;
- Fig. 1C: zeigt ein erstes Ausführungsbeispiel der vorliegenden Erfindung im betätigten Zustand von oben;
- Fig. 1D: zeigt ein erstes Ausführungsbeispiel der vorliegenden Erfindung im betätigten Zustand von der Seite;
- Fig. 2A: zeigt ein zweites Ausführungsbeispiel der vorliegenden Erfindung im unbetätigten Zustand von oben;
- Fig. 2B: zeigt ein zweites Ausführungsbeispiel der vorliegenden Erfindung im unbetätigten Zustand von der Seite;
- Fig. 2C: zeigt ein zweites Ausführungsbeispiel der vorliegenden Erfindung im betätigten Zustand von oben;
- Fig. 2D: zeigt ein zweites Ausführungsbeispiel der vorliegenden Erfindung im betätigten Zustand von der Seite;
- Fig. 3A: zeigt ein drittes Ausführungsbeispiel der vorliegenden Erfindung im unbetätigten Zustand von oben;
- Fig. 3B: zeigt ein drittes Ausführungsbeispiel der vorliegenden Erfindung im unbetätigten Zustand von der Seite;
- Fig. 3C: zeigt ein drittes Ausführungsbeispiel der vorliegenden Erfindung im betätigten Zustand von oben;
- Fig. 3D: zeigt ein drittes Ausführungsbeispiel der vorliegenden Erfindung im betätigten Zustand von der Seite;
- Fig. 4A: zeigt ein viertes Ausführungsbeispiel der vorliegenden Erfindung im unbetätigten Zustand von oben;
- Fig. 4B: zeigt ein viertes Ausführungsbeispiel der vorliegenden Erfindung im unbetätigten Zustand von der Seite;
- Fig. 4C: zeigt ein viertes Ausführungsbeispiel der vorliegenden Erfindung im betätigten Zustand von oben;
- Fig. 4D: zeigt ein viertes Ausführungsbeispiel der vorliegenden Erfindung im betätigten Zustand von der Seite;
- Fig. 5A: zeigt ein fünftes Ausführungsbeispiel der vorliegenden Erfindung im unbetätigten Zustand von oben;
- Fig. 5B: zeigt ein fünftes Ausführungsbeispiel der vorliegenden Erfindung im unbetätigten Zustand von der Seite;
- Fig. 5C: zeigt ein fünftes Ausführungsbeispiel der vorliegenden Erfindung im betätigten Zustand von oben;
- Fig. 5D: zeigt ein fünftes Ausführungsbeispiel der vorliegenden Erfindung im betätigten Zustand von der Seite; und
- Fig. 6: zeigt eine schematische Darstellung der Verformungsfigur gemäß dem vierten und fünften Ausführungsbeispiel.

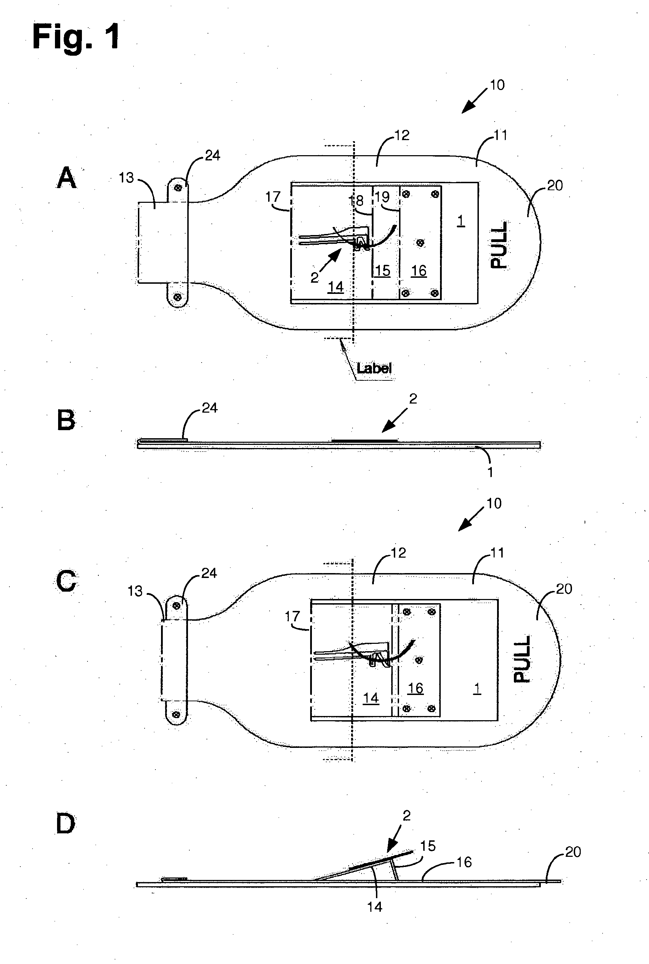

Ein erstes Ausführungsbeispiel der vorliegenden Erfindung ist unter Bezugnahme auf die Fig. 1A bis 1D im Detail beschrieben. Dieses erste Ausführungsbeispiel gehört zu dem ersten Aspekt der vorliegenden Erfindung.

Das erste Ausführungsbeispiel der vorliegenden Erfindung betrifft einen Nadel-Lift für chirurgisches Nahtmaterial mit zwei symmetrisch angeordneten, außen liegenden Zuglaschen 10 mit jeweils einem proximalen Ende 11, einem Mittelabschnitt 12 und einem distalen Ende 13. Die beiden Zuglaschen 10 sind im Bereich ihres Mittelabschnitts 12 zusammen geführt und im Bereich ihres distalen Endes 13 durch ein Brückenbauteil 24 an einer Grundfläche 1 verschiebbar gehalten. Das Brückenbauteil 24 verläuft quer über das distale Ende 13 der beiden zusammengeführten Zuglaschen 10 und ist beiderseits der zusammengeführten Zuglasche 10 an der Grundfläche 1 befestigt. Bei diesem Ausführungsbeispiel ist das Brückenbauteil 24 ein Teil der beiden zusammengeführten Zuglaschen 10 selbst. Das zusammengeführte distale Ende 13 der beiden Zuglaschen 10 ist umgefaltet, umgebogen bzw. umgedrückt und das Brückenbauteil 24 wird gebildet durch einen Bereich des distalen Endes 13, der breiter als der angrenzende Bereich der zusammengeführten Zuglaschen 10 ist. Das Brückenbauteil 24 liegt auf einem Teil der zusammengeführten Zuglasche 10.

Der Nadel-Lift gemäß diesem Ausführungsbeispiel weist zudem einen Knickabschnitt 14, 15, 16 auf, der mit seinem distale Ende an dem zusammengeführten Mittelabschnitt 12 der beiden Zuglaschen 10 befestigt ist und mit seinem proximalen Ende an einer Grundfläche 1 befestigt ist. Im vorliegenden Fall ist der Nadel-Lift an der Grundfläche fixiert, indem der Nadel-Lift punktuell auf die Grundfläche geschweißt wurde, beispielsweise durch Ultraschall-Schweißen. Es kann aber bei diesem und bei allen anderen Ausführungsbeispielen auch ein anderes Schweißverfahren verwendet werden, oder der Nadel-Lift kann durch Kleben, Nieten, Klemmen oder durch ein sonstiges Verfahren an der Grundfläche befestigt werden.

Der eine Knickabschnit 114, 15, 16 verfügt über drei Soll-Knickstellen 17, 18, 19, die quer zur Längsachse dem Zuglaschen 10 verlaufen. Eine Soll-Knickstelle 17 befindet sich genau an dem Übergang von dem distalen Ende des Knickabschnitts 14 zu dem zusammengeführten Mittelabschnitt 12 der Zuglaschen 10. Die Soll-Knickstelle 18 ist zwischen den beiden Knickabschnitten 14 und 15 angeordnet, welche die beiden Knickabschnitte sind, die sich von der Grundfläche 1 abheben können. Die Dritte Soll-Knickstelle 19 befindet sich zwischen dem Knickabschnitt 16 und dem an der Grundfläche 1 fixierten Knickabschnitt 16. An dem Knickabschnitt 14 ist eine Nadelhalterung 2 für eine chirurgische Nadel vorgesehen. Es wäre auch bei allen Ausführungsbeispielen möglich, zwei oder mehr Nadelhalterungen an dem Knickabschnitt 14 vorzusehen. Die eine Nadelhalterung 2 besteht aus einer Dreipunktlagerung, welche die Nadel so lagert, dass sie die mittlere Soll-Knickstelle 18 quert. Dreipunktlagerung bedeutet, dass die Nadel bei einer Betrachtung von oben auf einer Seite an einem Punkt gelagert ist und auf der anderen Seite an zwei Punkten. Da die Nadel gebogen ist, befindet sich der einzelne Lagerpunkt auf der Innenseite der Krümmung der Nadel und die beiden anderen Lagerpunkte befinden sich jeweils auf der Außenseite der Krümmung.

Die beiden Zuglaschen 10 verfügen über ein gemeinsames Griffelement 20, welches die beiden Zuglaschen 10 am proximalen Ende 11 verbindet. Im vorliegenden Fall ist das Griffelement 20 mit "pull" beschriftet, um die Handhabung für den Anwender zu erklären. Selbstverständlich können auch andere Hinweise an dem Griffelement 20 angebracht sein oder auch gar kein Hinweis.

In der noch nicht betätigten Stellung, wie sie in den Fig. 1A und 1B gezeigt ist, ist der Nadel-Lift sehr flach ausgebildet underhöht die Gesamtdicke einer Nahtmaterialverpackung nicht, da diese durch die Abmessungen des Fadenkanals bestimmt wird. Die gesamte Nahtmaterialverpackung ist wie eine bisherige Nahtmaterialverpackung inklusive Nahtmaterial steril in einer Umverpackung verpackt. Diese Umverpackung wird vom proximalen Ende her geöffnet und die Nahtmaterialverpackung wird aus der Umverpackung entnommen. Wenn der Operateur die Nadel des Nahtmaterials greifen möchte, zieht er oder eine sterile Schwester an dem Griffelement 20. Die Zugkraft wird über die beides symmetrischen Zuglaschen 10 zu dem Abschnitt übertragen, an dem die beiden Mittelabschnitte 12 der Zuglaschen 10 zusammengeführt sind und an dem das distale Ende des Knickabschnitts 14, 15, 16 angebracht ist. Das distale Ende 13 der Zuglaschen 10 schiebt also den Knickabschnitt 14 in Richtung des proximalen Endes 11. Dabei rutscht ein Teil des distalen Endes 13 unter dem Brückenbauteil 24 durch und die Schlaufe, die sich auf der distalen Seite des Brückenbauteils 24 befindet, wird teilweise unter das Brückenbauteil 24 gezogen. Gleichzeitig bilden sich an den Soll-Knickstellen 17, 18, 19 Knicke aus. Da der Knickabschnitt von unten durch die Grundfläche 1 gestützt ist, kann der Knickabschnitt 14, 15, 16 nur nach oben ausweichen, was er mit zunehmender Verschiebung des Griffabschnitts in proximaler Richtung auch tut. Wenn die Schlaufe auf der distalen Seite des Brückenbauteils 24 vollständig unter das Brückenbauteil 24 gezogen wurde, ist die maximale Verschiebung der Zuglaschen 10 erreicht und der Nadel-Lift hat seine in den Fig. 1C und 1D gezeigte Endposition erreicht.

Wie dies in der Fig. 1D gezeigt ist, bilden die beiden Knickabschnitte 14 und 15 eine Art schiefes Zeltdach, wobei die Seite des Knickabschnitts 15 kürzer aber steiler ist. Die Nadelhalterung 2 ist auf dem Knickabschnitt 14 so angeordnet, dass sich eine in dieser Nadelhalterung 2 gehaltene Nadel zu einem großen Teil über die Soll-Knickstelle 18 hinaus über den Knickabschnitt 15 erstreckt. Da dieser Knickabschnitt 15 relativ zu dem Knickabschnitt 14 nach unten weggeknickt ist, besteht nun ein sehr großzügiger Zugang zu der Nadel in der Nadelhalterung 2. Der Operateur kann nun die nadel ganz leicht mit einem Nadelhalter aufnehmen.

Wie dies ebenfalls in den Fig. 1C und 1D gezeigt ist, verbleibt die Nadelspitze auch während der Betätigung des Nadel-Lifts unterhalb des Beschriftungsbauteils 28, welches das Label der Nahtmaterialverpackung ist, auf dem die relevanten Informationen zum Nahtmaterial, zum Hersteller, etc. angegeben sind.

Bei diesem Ausführungsbeispiel ist keine Einrichtung vorgesehen, welche den Nadel-Lift in der Betätigten Stellung hält. Es könnte jedoch das Brückenbauteil 24 als ein Reibelement ausgebildet werden, um diese Funktion zu erfüllen. Denkbar ist auch, dass die plastische Verformung des distalen Endes 13 der Zuglaschen, während sie unter dem Brückenbauteil 24 hindurch gezogen werden, solche eine Einrichtung bilden, da eine erneute plastische Verformung dieses Abschnitts erforderlich wäre, um den Nadel-Lift in die Ausgangsposition zurück zu führen.

Hat der Operateur die Nadel gegriffen, kann er den Faden des Nahtmaterials einfach aus dem Fadenkanal ziehen. Damit dies einfach und fehlerfrei funktioniert, gibt es im Stand der Technik zahlreiche Ausführungsformen für Fadenkanäle.

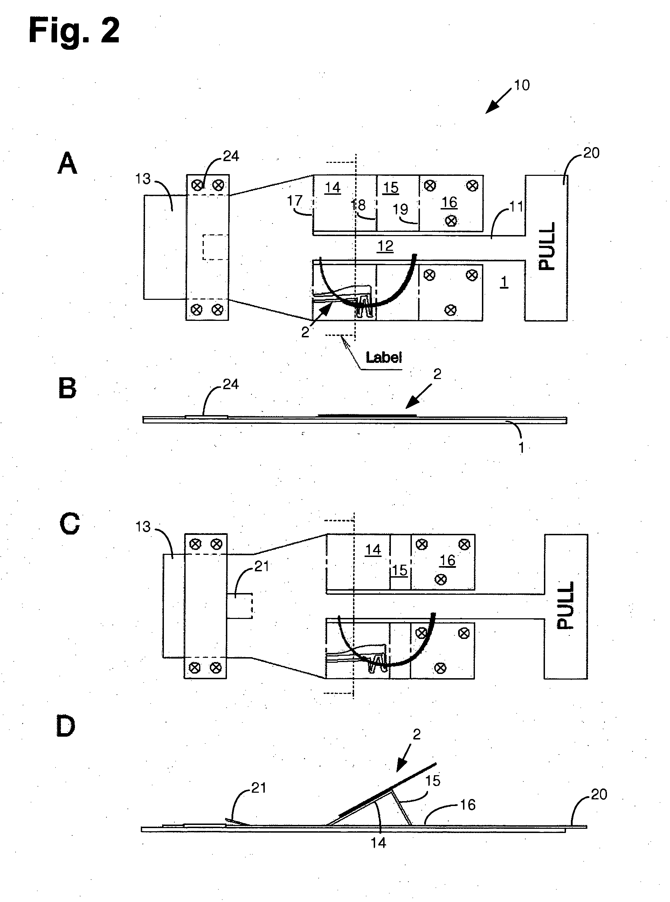

Ein zweites Ausführungsbeispiel der vorliegenden Erfindung ist im Folgenden unter Bezugnahme auf die Fig. 2A bis 2D beschrieben. Das zweite Ausführungsbeispiel bezieht sich ebenfalls auf den zweiten Aspekt der vorliegenden Erfindung.

Das zweite Ausführungsbeispiel hat viele Gemeinsamkeiten mit dem ersten Ausführungsbeispiel und dieselbe Funktionsweise. Daher werden hier lediglich die Unterschiede zum ersten Ausführungsbeispiel erklärt. Das zweite Ausführungsbeispiel verfügt über eine einzelne zentrale Zuglasche 10. Zwei Knickabschnitte 14, 15, 16 sind seitlich der Zuglasche 10 angeordnet und jeweils mit ihrem distalen Ende an dem Mittelabschnitt 12 der Zuglasche 10 angebracht. Im vorliegenden Ausführungsbeispiel ist nur an einem der Knickabschnitte 14 eine Nadelhalterung 2 vorgesehen, es können aber auch an beiden Knickabschnitten 14 eine oder mehrere Nadelhalterungen 2 vorgesehen sein.

Das distale Ende 13 der Zuglasche 10 ist frei. Ein gesondertes Brückenbauteil 24 verläuft in der Nähe des distalen Endes 13 der Zuglasche 10 quer über die Zuglasche 10 und hält so das distale Ende 13 und den Mittelabschnitt 12 der Zuglasche 10 sowie das distale Ende der beiden Knickabschnitte 14 in der Nähe der Grundfläche 1. Außerdem verfügt der Mittelabschnitt 12 der Zuglasche 10 über einen Vorsprung 21, der ausgebildet wird, indem ein U-förmiger Spalt in den Mittelabschnitt 12 gestanzt wird, der zur proximalen Seite hin offen ist, und anschließend die sich ergebende Lasche von unten so weit nach oben gebogen wird, bis die Verbindungsstelle leicht plastiziert, sodass sich die Lasche nicht wieder komplett in ihre Ausgangslage zurückverformt. Eine gleichwertige Halteeinrichtung 21 kann auch auf andere Weise hergestellt werden, beispielsweise indem ein weiteres Bauteil an dem Mittelabschnitt 12 angebracht wird oder der Mittelabschnitt 12 mit Hilfe von z.B. Wärme lokal verformt wird. Wie dies in der Fig. 2D gezeigt ist, springt die Lasche 21, welche die Halteeinrichtung 21 bildet, leicht nach oben, sobald sie das Brückenbauteil 24 unterquert hat. Dann liegt die Lasche 21 an der Seitenfläche des Brückenbauteils 24 an und verhindert, dass der Nadel-Lift in seine Ausgangsposition zurück kehrt. Zusätzlich dazu ist noch eine Halteeinrichtung 22 in Form zweier lateraler Vorsprünge an dem distalen Ende 13 der Zuglasche 10 vorgesehen. Diese passieren unter einer gewissen elastischen Verformung, somit also auch einem gewissen Kraftaufwand, die erste Verbindungsstelle zwischen Brückenbauteil 24 und Grundfläche 1. Zwischen der ersten und der zweiten Verbindungsstelle des Brückenbauteils 24 kommen die Halteeinrichtungen 22 zum liegen. Die zweite Verbindungsstelle dient somit auch als Begrenzung der Verschiebung der Zuglasche 10 in Richtung zu dem proximalen Ende 11 hin und gleichzeitig als Halteeinrichtung, um ein Zurückkehren des Nadel-Lifts in die Ausgangsposition zu verhindern. Auf die Halteeinrichtung 21 könnte bei diesem Ausführungsbeispiel also auch verzichtet werden.

Wie zuvor bereits erwähnt ist die Funktionsweise dieses Ausführungsbeispiels gleich der des ersten Ausführungsbeispiels. Durch ziehen an dem Griffelement 20 wird das distale Ende der Zuglasche 10 und damit auch das distale Ende des Knickabschnitts 14, 17 zu dem proximalen Ende des Knickabschnitts 16 hin verschoben, wodurch sich der distale und mittlere Knickabschnitt 14, 15 von der Grundfläche 1 abheben und so einen leichten Zugang zu der Nadel in dem Nadelhalter 2 ermöglichen.

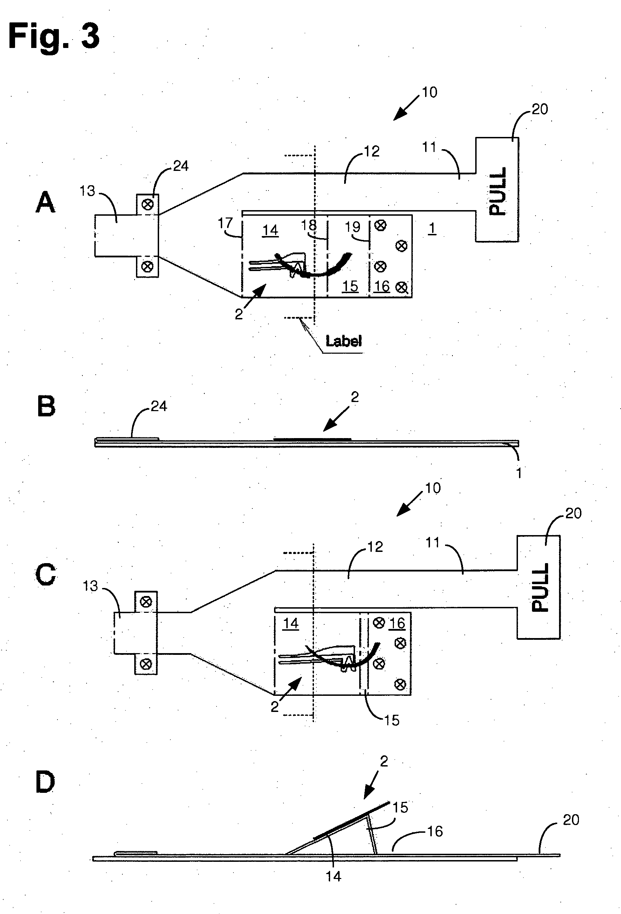

Ein drittes Ausführungsbeispiel der vorliegenden Erfindung ist im Folgenden unter Bezugnahme auf die Fig. 3A bis 3D beschrieben. Das dritte Ausführungsbeispiel bezieht sich ebenfalls auf den zweiten Aspekt der vorliegenden Erfindung.

Im Unterschied zum ersten und zweiten Ausführungsbeispiel ist das dritte Ausführungsbeispiel nicht symmetrisch aufgebaut. Genauer gesagt weist dass dritte Ausführungsbeispiel einen Zugabschnitt 10 und einen Knickabschnitt 14, 15, 16 auf. Das distale Ende der Zuglasche 10 ist wie beim ersten Ausführungsbeispiel aufgebaut. Die Funktionsweise des dritten Ausführungsbeispiels entspricht der des ersten und zweiten Ausführungsbeispiels.

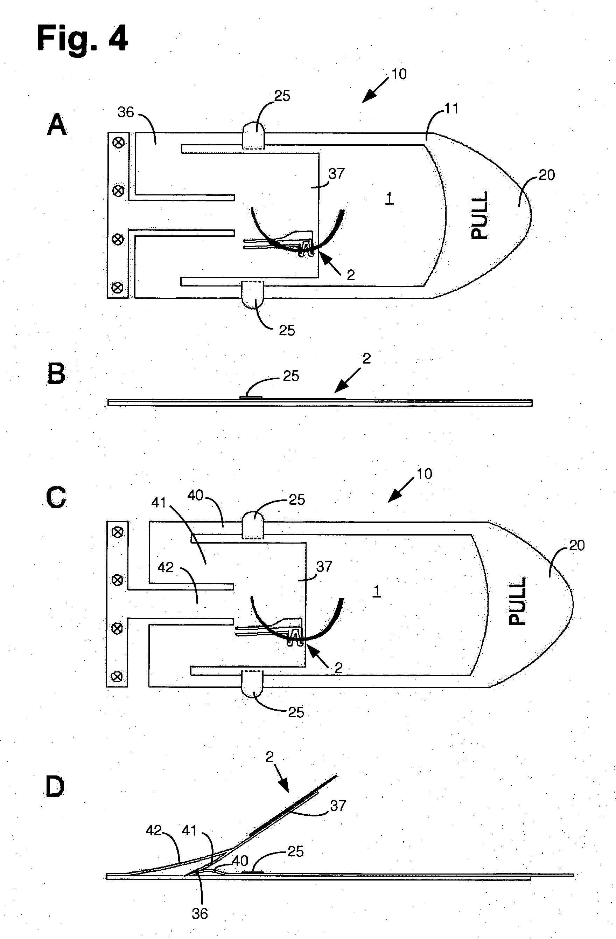

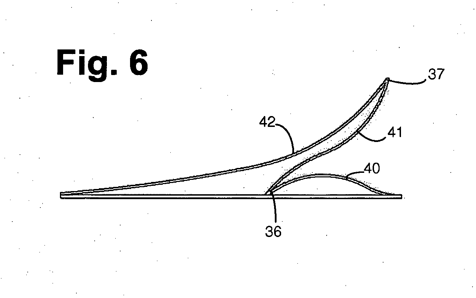

Ein viertes Ausführungsbeispiel der vorliegenden Erfindung ist im Folgenden unter Bezugnahme auf die Fig. 4A bis 4D und Fig. 6 beschrieben. Das vierte Ausführungsbeispiel bezieht sich auf den dritten Aspekt der vorliegenden Erfindung.

Gemäß diesem Ausführungsbeispiel sind zwei Zuglaschen 10 an ihrem proximalen Ende 11 durch ein Griffelement 20 verbunden. Im Bereich der beiden Mittelabschnitte 12 der beiden Zuglaschen 10 ist jeweils ein "S"-förmiger Abschnitt spiegelsymmetrisch ausgebildet. Dabei geht das proximale Ende 11 einer Zuglasche in eine distale Biegung 36 des "S"-förmigen Abschnitts über. An diesen schließt sich die proximale Biegung 37 an. Die beiden proximalen Biegungen 37 der beiden Zuglaschen 10 sind mittig miteinander verbunden. An die proximalen Biegungen 37 schließt sich ein distaler Haltebereich an, an dem die Zuglaschen 10 an der Grundfläche 1 fixiert sind. Ein Nadelhalter 2 ist im Bereich der beiden proximalen Biegungen an dem Mittelabschnitt 12 der Zuglaschen 10 vorgesehen. Der Nadelhalter ist so gestaltet und an dem Mittelabschnitt 12 der Zuglaschen 10 angeordnet, dass sich eine durch den Nadelhalter 2 gehaltene Nadel teilweise über die proximale Biegung 37 hinaus in Richtung zu dem proximalen Ende 11 erstreckt. Zudem sind in der Grundfläche 1 Laschen 25 ausgebildet, die mit dem mittleren Abschnitt 12 der Zuglaschen 10 eingreifen. Im vorliegenden Fall weisen, wie dies aus der Fig. 4A ersichtlich ist, die kragarmförmigen Laschen 25 so ausgebildet, dass ihr freies Ende lateral nach außen vorsteht und die die Zuglaschen 10 im Bereich vor der distalen Biegung 36 von innen greifen.

Die Laschen können aber auch so ausgebildet sein, dass ihr freies Ende lateral nach innen liegt und sie den Mittelabschitt 12 der Zuglaschen 10 von innen greifen. Die Laschen sind dazu vorgesehen, die distalen Biegungen 36 der Zuglaschen 10 in der Nähe der Grundfläche 1 zu halten.

Wird nun an dem Griffelement 20 gezogen, werden die distalen Biegungen 36 in proximaler Richtung verschoben. Dadurch kommt es in den "S"-förmigen Abschnitten zu einer Stauchung. Um diese Stauchung auszugleichen, werden sich entweder die distalen Biegungen 36 oder die proximalen Biegungen 37 von der Grundfläche 1 abheben. Da aber die distalen Biegungen 36 durch die Laschen 25 in der Nähe der Grundfläche 1 gehalten sind, werden sich die proximalen Biegungen 37 von der Grundfläche 1 abheben. Dabei biegt sich der Mittelabschnitt 12 der Zuglaschen 10. Sollknickstellen sind bei dem vorliegenden Ausführungsbeispielen nicht ausgebildet, es können aber auch Soll-Knickstellen im Zusammenhang mit dieser technischen Lehre eingesetzt werden. Ohne Soll-Knickstellen werden die "S"-förmigen Abschnitte 36, 37 aus der Ebene der Grundfläche 1 heraus verbogen, wie dies aus Fig. 4D ersichtlich ist. Eine exakte Beschreibung der Verformung ist im Folgenden für eine der beiden symmetrischen Zuglaschen 10 gegeben, nämlich für die in der Fig. 4A obere Zuglasche 10. Dazu wird der obere "S"-förmige Abschnitt der Zuglasche für eine einfachere Beschreibung in einen äußeren Schenkel 40, einen mittleren Schenkel 41 und einen inneren Schenkel 42 aufgegliedert. Die beiden "S"-förmigen Abschnitte sind demnach an ihren inneren Schenkeln 42 miteinander verbunden. Das Griffelement 20 sowie der proximale Teil der Zuglasche 10 bis zu der Lasche 25, mit der die Zuglasche an der Grundfläche 1 gehalten wird, bleibt im Wesentlichen eben auf der Grundfläche 1 liegen. Allerdings kann sich auch dieser Teil der Zuglasche zu einem gewissen Grad aufgrund von Verwindungen und Sekundärkräften leicht von der Grundfläche 1 abheben. Dies hat allerdings keinen Einfluss auf die Funktionsweise oder Funktionsfähigkeit der vorliegenden Erfindung. Distal von der Lasche 25 bildet sich in dem äußeren Schenkel 40 ein konvexer Abschnitt aus. Das distale Ende der distalen Biegung 36 befindet sich in Kontakt mit der Grundfläche 1, gegen welche die distale Biegung 36 gedrückt wird. Zwischen distalem Ende der distalen Biegung und proximalem Ende der proximalen Biegung, also in dem mittleren Schenkel 41, bildet sich entsprechend der Ansicht der Fig. 4D ein "S"-förmig gebogener Bereich aus. Von distaler Biegung 36 zu proximaler Biegung 37 bilden sich zuerst ein konvexer Biegebereich und anschließend ein konkaver Biegebereich aus. Der innere Schenkel 42 wiederum wird konkav gebogen. An der distalen Biegung 36 werden somit der äußere Schenkel 40 und der mittlere Schenkel 41 konvex gebogen, wobei der mittlere Schenkel 41 einen kleineren Krümmungsradius erhält. An der proximalen Biegung 37 werden der mittlere Schenkel 41 und der innere Schenkel 422 konkav gebogen, wobei wiederum der mittlere Schenkel 41 einen kleineren Krümmungsradius als der innere Schenkel 42 erhält. Dies ist auch ersichtlich aus der Fig. 6, welche die Verformungsfigur des vierten und fünften Ausführungsbeispiels schematisch zeigt. Die Verformung ist in der Fig. 6 zur besseren Sichtbarkeit deutlich überzeichnet.

Der Nadelhalter 2 ist im Bereich der proximalen Biegung 37 so angeordnet, dass sich eine darin gehaltene Nadel mit dem Ende, an dem der Faden angebracht ist, über das proximale Ende der proximalen Biegung 37 erstreckt. Bei betätigtem Nadel-Lift entsprechen diesem Ausführungsbeispiel, das heißt wenn sich vorstehend beschriebene Verformungsfigur eingestellt hat, ist der hintere Teil der Nadel so von der Grundfläche 1 abgehoben, dass er leicht mit einem Nadelhalter gegriffen werden kann. Es können auch bei diesem Ausführungsbeispiel mehrere Nadelhalter 2 vorgesehen sein.

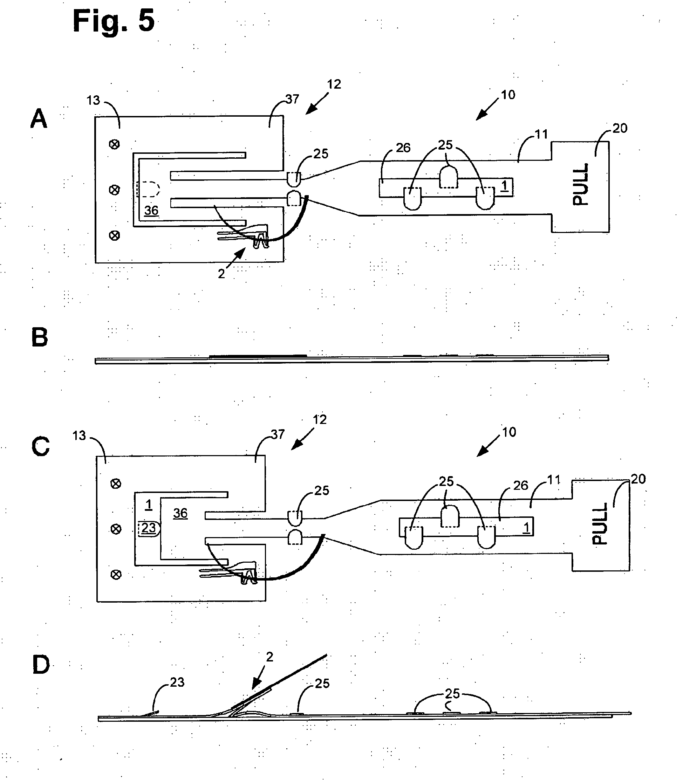

Ein fünftes Ausführungsbeispiel der vorliegenden Erfindung ist im Folgenden unter Bezugnahme auf die Fig. 5A bis 5D und Fig. 6 beschrieben. Das fünfte Ausführungsbeispiel bezieht sich auch auf den dritten Aspekt der vorliegenden Erfindung. Das fünfte Ausführungsbeispiel verhält sich zum vierten Ausführungsbeispiel in etwa so wie das zweite Ausführungsbeispiel sich zum ersten Ausführungsbeispiel verhält.

Das fünfte Ausführungsbeispiel unterscheidet sich von dem vierten Ausführungsbeispiel dahingehend, dass die beiden "S"-förmigen Abschnitte in ihrer Position vertauscht sind. Genauer gesagt befanden sich bei dem vierten Ausführungsbeispiel die distalen Biegungen 36 seitlich außerhalb der proximalen Biegungen 37 und die beiden "S"-förmigen Abschnitte waren im Bereich der proximalen Biegungen 37 miteinander verbunden. Beim fünften Ausführungsbeispiel befinden sich die beiden proximalen Biegungen 37 seitlich außerhalb der distalen Biegungen 36 und die beiden "S"-förmigen Abschnitte sind im Bereich der distalen Biegungen 36 miteinander verbunden. Die Zuglasche 10 ist hier mit den inneren Schenkeln 42 der "S"-förmigen Abschnitte verbunden. Die Zuglasche 10 ist im Bereich des proximalen Endes 11 mit einem Schlitz 26 versehen. Durch diesen hindurch erstrecken sich Laschen 25 derart, dass die Zuglasche 10 sich zwischen den freien Enden der Laschen 25 und der Grundfläche 1 befindet. Bei diesem Ausführungsbeispiel erstrecken sich die kragarmförmigen Laschen 25 wechselweise in beide seitlichen Richtungen der Zuglasche 10. Im Bereich der inneren Schenkel 42 ist die Zuglasche 10 zudem durch weitere Laschen 25, welche sich von außen zur Mitte hin erstrecken, in der Nähe der Grundfläche 1 gehalten. Auf diese Weise wird die Verformungsfigur so bestimmt, dass sich die proximale Biegungen 37 von der Grundfläche abheben und die distalen Biegungen 36 gegen die Grundfläche 1 gedrückt werden. Bei einer seitlichen Betrachtung entsprechend der Fig. 5D ergibt sich wiederum eine Verformungsfigur entsprechend der Fig. 6. Allerdings ist in diesem Fall die inneren Schenkel 42 und die äußeren Schenkel 40 vertauscht, was in der Fig. 6 nicht erkennbar ist.

Die Fig. 6 unterscheidet sich von den Ausführungsformen der Fig. 4 und 5 zudem darin, dass die beiden Biegungen 36 und 37 sehr schmal ausgebildet sind, sodass sich in der Fig. 6 bereits ein Winkel zwischen den Bereichen 40 und 41 bzw. 41 und 42 bildet.

darüber hinaus verfügt das fünfte Ausführungsbeispiel über eine Einrichtung 23, welche den Nadel-Lift in der betätigten Stellung hält. Diese Einrichtung wird durch eine weitere kragarmförmige Lasche 23 gebildet, welche im Bereich der distalen Biegungen 36 angeordnet ist. Die Lasche 23 kann so ausgebildet sein wie die Lasche 21 aus dem zweiten Ausführungsbeispiel. Nachdem aber bei den Ausführungsbeispielen gemäß dem dritten Aspekt der vorliegenden Erfindung, also dem vierten und fünften Ausführungsbeispiel, die distale Biegung bzw. distalen Biegungen 36 durch die Verformung gegen die Grundfläche 1 gedrückt wird bzw. werden, kann die Einrichtung 23 auch durch einen einfachen Vorsprung gebildet sein. Dieser verhindert lediglich, dass bei einer Entlastung der Zuglasche 10 durch Freigeben des Griffelements 20 die distale(n) Biegung(en) entlang der Grundfläche 1 in ihre Ausgangsposition(en) zurückkehrt bzw. zurückkehren.

Weitere Kombinationen der einzelnen Merkmale sind möglich und dem Fachmann ergeben sich aus dieser Beschreibung und den beigefügten Ansprüchen und Figuren zahlreiche weitere Modifikationen und Abwandlungen.

Prinzipiell sind einzelne Aspekte der verschiedenen Ausführungsbeispiele miteinander austauschbar und kombinierbar. So können zum Beispiel die Brückenbauteile und die Laschen, welche beide die Funktion haben, das entsprechend gehaltene Bauteil in der Nähe der Grundfläche 1 zu halten, beliebig gegeneinander ausgetauscht werden oder aber auch gemeinsam an einem Ausführungsbeispiel eingesetzt werden. Bei den ersten beiden Ausführungsbeispielen kann eine oder mehr Soll-Knickstellen durch Biegestellen ersetzt werden. Eine Soll-Knickstelle kann durch eine Einkerbung oder durch eine Perforation oder andere Mittel gekennzeichnet sein. Eine Biegestelle kann auch eine Soll-Biegestelle (bzw. Soll-Biegebereich) sein, an der die Dicke des Bauteils gegenüber der Umgebung über einen gewissen Bereich in Längsrichtung des Nadellifts verringert ist. Bei dem dritten und vierten Ausführungsbeispiel kann auch ein oder mehr Biegebereich durch eine Soll-Knickstelle ersetzt werden.

Ein Brückenbauteil kann durch ein Element eines Deckelbauteils oder aber eines Beschriftungsbauteils einer Nahtmaterialverpackung gebildet sein. Die Grundfläche 1 kann sich an einem Deckelbauteil oder an einem Bodenbauteil ausgebildet sein, je nachdem, wie die Nahtmaterialverpackung aufgebaut ist. Beispiele hierfür finden sich im Stand der Technik. Jedes Ausführungsbeispiel kann mit einer oder mehreren Nadelhalterungen ausgebildet sein, die Zuglaschen können aus verschiedenen Materialien bestehen und verschiedene Beschriftungen aufweisen. Bei allen Ausführungsbeispielen ist es vorteilhaft, wenn sich die Nadelspitzen auch nach Betätigung des Nadel-Lifts noch bedeckt sind, vorzugsweise durch ein Beschriftungsbauteil. Dieses kann auch verformbar sein, insbesondere biegbar, und sich bei einer Betätigung des Nadel-Lifts teilweise von dem Deckelbauteil wegbiegen, wie dies in der Fig. 1D gezeigt ist.

Alle Ausführungsbeispiele können Rückhalteeinrichtungen aufweisen, die den Nadel-Lift in der betätigten Stellung halten. Dazu eignen sich Vorsprünge und Laschen, die dem Prinzip nach wie die Laschen 21 und/oder 23 funktionieren. Solche Laschen, die ein Rückhalten aufgrund Formschluss zwischen beweglichem Teil des Nadel-Lifts und unbeweglichem Teil der Nahtmaterialverpackung gewährleisten, können nicht nur an der Grundfläche ausgebildet sein sondern auch an dem Beschriftungsbauteil. Es kann aber auch eine kraftschlüssige Rückhalteeinrichtung realisiert werden. Ein Beispiel hierfür ist im ersten Ausführungsbeispiel gezeigt, bei dem ein Brückenbauteil gegen einen Teil der Zuglasche 10 drückt. Es kann aber auch ein elastisches Bauteil, beispielsweise ein Schaumgummi, zwischen Bodenbauteil, Deckelbauteil bzw. Beschriftungsbauteil und beweglichem Teil des Nadel-Lifts geklemmt werden, welches eine ausreichende Reibung auf den Nadel-Lift aufbringt, um diesen in der betätigten Stellung zu halten. Aber auch die Rückhaltung mittels Rastvorsprüngen 22 gemäß dem zweiten Ausführungsbeispiel kann auf alle anderen Ausführungsbeispiele angewandt werden.

## Patentansprüche

1. Nadel-Lift für chirurgisches Nahtmaterial mit:
einer Grundfläche (1), und
einem bewegbaren Bauteil (10), das zumindest teilweise gegenüber der Grundfläche (1) in der Ebene der Grundfläche (1) bewegbar ist,
wobei das bewegbare Bauteil (10) so geformt und an der Grundfläche (1) befestigt ist, dass eine Bewegung des bewegbaren Bauteils (10) in der Ebene der Grundfläche (1) dazu führt, dass sich zumindest ein Bereich (14, 15) des bewegbaren Bauteils (10) von der Grundfläche (1) abhebt,
**dadurch gekennzeichnet, dass**
das bewegbare Bauteil (10) mindestens eine Zuglasche (10) mit einem proximalen Ende (11), einem Mittelabschnitt (12) und einem distalen Ende (13) aufweist, wobei die mindestens eine Zuglasche (10) im Bereich ihres distalen Endes (13) an der Grundfläche (1) verschiebbar gehalten ist, und
mindestens ein Knickabschnitt (14, 15, 16) vorgesehen ist, der mit seinem distalen Ende an dem Mittelabschnitt (12) der mindestens einen Zuglasche (10) befestigt ist und mit seinem proximalen Ende an der Grundfläche (1) befestigt ist,
wobei an dem mindestens einen Knickabschnitt (14, 15, 16) eine Nadelhalterung (2) für eine chirurgische Nadel vorgesehen ist, und
wobei ein Ziehen der Zuglasche (10) ein Abheben der Nadelhalterung (2) von der Grundfläche (1) bewirkt.

2. Nadel-Lift für chirurgisches Nahtmaterial nach Anspruch 1,
wobei der mindestens eine Knickabschnitt (14, 15, 16) über wenigstens eine Soll-Knickstelle (17, 18, 19) verfügt, die im Wesentlichen quer zur Längsachse der mindestens einen Zuglasche (10) verläuft.

3. Nadel-Lift für chirurgisches Nahtmaterial nach Anspruch 1 oder 2,
wobei
der mindestens eine Knickabschnitt (14, 15, 16) über drei Soll-Knickstellen (17, 18, 19) verfügt und die Nadelhalterung (2) aus einer Dreipunktlagerung besteht, welche die Nadel so lagert, dass sie die mittlere Soll-Knickstelle (18) quert.

4. Nadel-Lift für chirurgisches Nahtmaterial mit:
einer Grundfläche (1), und
einem bewegbaren Bauteil (10), das zumindest teilweise gegenüber der Grundfläche (1) in der Ebene der Grundfläche (1) bewegbar ist,
wobei das bewegbare Bauteil (10) so geformt und an der Grundfläche (1) befestigt ist, dass eine Bewegung des bewegbaren Bauteils (10) in der Ebene der Grundfläche (1) dazu führt, dass sich zumindest ein Bereich (14, 15) des bewegbaren Bauteils (10) von der Grundfläche (1) abhebt,
**dadurch gekennzeichnet, dass**
das bewegbare Bauteil (10) mindestens eine Zuglasche (10) mit einem proximalen Ende (11), einem Mittelabschnitt (12) und einem distalen Ende (13) aufweist, wobei die mindestens eine Zuglasche (10) im Bereich ihres distalen Endes (13) an der Grundfläche (1) befestigt ist und die mindestens eine Zuglasche (10) im Bereich ihres proximalen Endes (11) an der Grundfläche (1) verschiebbar gehalten ist,
wobei der Mittelabschnitt (12) als mindestens ein "S"-förmiger Abschnitt ausgebildet ist, welcher das proximale Ende (11) und das distale Ende (13) der Zuglasche (10) verbindet,
wobei an dem mindestens einen "S"-förmigen Abschnitt eine Nadelhalterung (2) für eine chirurgische Nadel vorgesehen ist, und
wobei ein Ziehen der Zuglasche (10) ein Abheben der Nadelhalterung (2) von der Grundfläche (1) bewirkt.

5. Nadel-Lift für chirurgisches Nahtmaterial nach Anspruch 4,
wobei die Nadelhalterung (2) aus einer Dreipunktlagerung besteht, welche die Nadel so lagert, dass sich das der Nadelspitze entgegengesetzte Ende der Nadel außerhalb des "S"-förmigen Bereichs zu dem proximalen Ende (11) der mindestens einen Zuglasche (10) hin befindet.

6. Nadel-Lift für chirurgisches Nahtmaterial nach einem der Ansprüche 1 bis 5,
wobei die mindestens eine Zuglasche (10) über ein Griffelement (20) verfügt.

7. Nadel-Lift für chirurgisches Nahtmaterial nach einem der Ansprüche 1 bis 6,
wobei die mindestens eine Zuglasche (10) und/oder die Grundfläche (1) über eine Einrichtung (21, 22, 23) verfügt, welche die mindestens eine Zuglasche (10) nach einer Verschiebung derselben in der verschobenen Stellung hält.

8. Nadel-Lift für chirurgisches Nahtmaterial nach einem der Ansprüche 1 bis 7,
wobei die mindestens eine Zuglasche (10) an der Grundfläche (1) verschiebbar gehalten ist, indem ein Brückenbauteil (24) über die mindestens eine Zuglasche (10) verläuft, welches beiderseits der mindestens einen Zuglasche (10) an der Grundfläche (1) befestigt ist, wobei das Brückenbauteil (24) vorzugsweise ein Teil der mindestens einen Zuglasche (10) selbst ist, welches umgefaltet, umgebogen bzw. umgeknickt ist, breiter als der angrenzende Bereich der mindestens einen Zuglasche (10) ist, und auf einem Teil der mindestens einen Zuglasche (10) liegt.

9. Nadel-Lift für chirurgisches Nahtmaterial nach einem der Ansprüche 1 bis 8,
wobei die mindestens eine Zuglasche (10) an der Grundfläche (1) verschiebbar gehalten ist, indem in der Grundfläche (1) kragarmförmige Laschen (25) ausgebildet sind, welche beiderseits der mindestens einem Zuglasche (10) angeordnet sind, wobei die mindestens eine Zuglasche (10) zwischen den freien Enden der kragarmförmigen Laschen (25) und der Grundfläche (1) angeordnet ist.

10. Nadel-Lift für chirurgisches Nahtmaterial nach einem der Ansprüche 1 bis 9,
wobei die mindestens eine Zuglasche (10) an der Grundfläche (1) verschiebbar gehalten ist, indem in der mindestens einen Zuglasche (10) ein Schlitz (26) entlang ihrer Längsachse vorgesehen ist und in der Grundfläche (1) kragarmförmige Laschen (25) ausgebildet sind, welche sich in beide Richtungen quer zur Längsachse der mindestens einen Zuglasche (10) durch den Schlitz (26) erstrecken, und wobei die mindestens eine Zuglasche (10) zwischen den freien Enden der kragarmförmigen Laschen (25) und der Grundfläche (1) angeordnet ist.

11. Nahtmaterialverpackung mit:
einem Bodenbauteil, und
einem Deckelbauteil, wobei zwischen Bodenbauteil und Deckelbauteil zumindest teilweise ein Aufnahmeraum für den Faden ausgebildet ist,
einer Grundfläche (1), welche an dem Bodenbauteil oder dem Deckelbauteil vorgesehen ist, und
einem Nadel-Lift nach einem der vorstehenden Ansprüche.

12. Nahtmaterialverpackung nach Anspruch 11, wobei
die Grundfläche (1) an dem Bodenbauteil vorgesehen ist und der Nadel-Lift sich zwischen Bodenbauteil und Deckelbauteil befindet, sodass die Bereiche der Zuglasche (10), welche gegenüber der Grundplatte (1) verschiebbar gehalten sind, durch das Deckelbauteil gehalten sind.

13. Nahtmaterialverpackung nach Anspruch 11 oder 12, wobei
ein Beschriftungsbauteil auf dem Deckelbauteil vorgesehen ist, welches zumindest die Nadelspitze auch dann noch abdeckt, wenn der Nadel-Lift betätigt wurde.

## Claims

1. Needle lift for surgical suturing material having:
a base surface (1), and
a movable component (10), which is at least partially movable relative to the base surface (1) in the plane of the base surface (1),
wherein the movable component (10) is so shaped and attached to the base surface (1) that a movement of the movable component (10) in the plane of the base surface (1) results in that at least one area (14, 15) of the movable component (10) lifts off the base surface (1),
**characterized in that**
the movable component (10) exhibits at least one tensioning loop (10) with a proximal end (11), a center section (12) and a distal end (13), wherein the at least one tensioning loop (10) in the area of its distal end (13), is held so as to shift on the base surface (1), and
at least one bending section (14, 15, 16) is provided, which is attached to the center section (12) of the at least one tensioning loop (10) with its distal end, and which is attached to the base surface (1) at its proximal end,
wherein on the at least one bending section (14, 15, 16) a needle holder (2) is provided for a surgical needle, and
wherein pulling on the tensioning loop (10) causes the needle holder (2) to lift away from the base surface (1).

2. Needle lift for surgical suturing material according to claim 1, wherein the at least one bending section (14, 15, 16) has at least one target bending position (17, 18, 19), which essentially runs transversely to the longitudinal axis of the at least one tensioning loop (10).

3. Needle lift for surgical suturing material according to claim 1 or 2, wherein the at least one bending section (14, 15, 16) has three target bending positions (17, 18, 19) and the needle holder (2) consists of a three-point bearing, which supports the needle so that it crosses the center target bending position (18).

4. Needle lift for surgical suturing material having:
a base surface (1), and
a movable component (10), which is at least partially movable relative to the base surface (1) in the plane of the base surface (1),
wherein the movable component (10) is shaped and attached to the base surface (1) in such a manner that a movement of the movable component (10) in the plane of the base surface (1) results in that at least one area (14, 15) of the movable component (10) lifts off the base surface (1),
**characterized in that**
the movable component (10) exhibits at least one tensioning loop (10) with a proximal end (11), a center section (12) and a distal end (13), wherein the at least one tensioning loop (10) is attached to the base surface (1) in a movable manner in the area of its distal end (13),
wherein the center section (12) is configured as at least one "S" shaped section, which connects the proximal end (11) and the distal end (13) of the tensioning loop (10) wherein, on the at least one "S" shaped section, a needle holder (2) is provided for a surgical needle, and
wherein pulling on the tensioning loop (10) causes the needle holder (2) to lift away from the base surface (1).

5. Needle lift for surgical suturing material according to claim 4,
wherein the needle holder (2) consists of a three-point bearing, which supports the needle so that the end of the needle opposite the needle tip, is located outside the "S" shaped area, toward the proximal end (11) of the at least one tensioning loop (10).

6. Needle lift for surgical suturing material according to one of claims 1 to 5, wherein the at least one tensioning loop (10) has a gripping element (20).

7. Needle lift for surgical suturing material according to one of claims 1 to 6, wherein the at least one tensioning loop (10) and/or the base surface (1) has a device (21, 22, 23), which holds the at least one tensioning loop (10) in the shifted position after shifting the same.

8. Needle lift for surgical suturing material according to one of claims 1 to 7, wherein the at least one tensioning loop (10) is held so as to shift on the base surface (1), in that a bridge piece (24) runs over the at least one tensioning loop (10), which is attached on both sides of the at least one tensioning loop (10) to the base surface (1), wherein the bridge piece (24) preferably is part of the at least one tensioning loop (10) itself, which is folded over, bent or creased, is wider than the adjoining area of the at least one tensioning loop (10), and lies on a part of the at least one tensioning loop (10).

9. Needle lift for surgical suturing material according to one of claims 1 to 8, wherein the at least one tensioning loop (10) is held so as to shift on the base surface (1), in that cantilever-shaped loops (25) are formed in the base surface (25), which loops are arranged on both sides of the at least one tensioning loop (10), wherein the at least one tensioning loop (10) is situated between the free ends of the cantilever-shaped loops (25) and the base surface (1).

10. Needle lift for surgical suturing material according to one of claims 1 to 9, wherein the at least one tensioning loop (10) is held so as to shift on the base surface (1), in that, in the at least one tensioning loop (10), a slot (26) is provided along its longitudinal axis, and in the base surface (1), cantilever-shaped loops (25) are formed, which extend in both directions transversely to the longitudinal axis of the at least one tensioning loop (10) through the slot (26), and wherein the at least one tensioning loop (10) is arranged between the free ends of the cantilever-shaped loops (25) and the base surface (1).

11. Suturing material packaging with
a bottom section, and
a cover section, wherein between the bottom section and the cover section, at least partially an intake space for the thread is formed,
a base surface (1), which is provided on the bottom section or the cover section, and
a needle lift according to one of the preceding claims.

12. Suturing material packaging according to claim 11, wherein the base surface (1) is provided on the bottom section and the needle lift is located between the bottom section and the cover section, so that the areas of the tensioning loop (10) which are held so as to shift relative to the base plate (1) are held by the cover section.

13. Suturing material according to claim 11 or 12, wherein an inscription component is provided on the cover section, which at least covers the needle tip even when the needle lift is actuated.

## Revendications

1. Lève-aiguille pour matériel de suture chirurgicale, comprenant :
une surface de base (1) et
un composant mobile (10), qui est mobile au moins partiellement par rapport à la surface de base (1) dans le plan de la surface de base (1),
dans lequel le composant mobile (10) est formé et fixé à la surface de base (1) de telle sorte qu'un mouvement du composant mobile (10) dans le plan de la surface de base (1) entraîne qu'au moins une zone (14, 15) du composant mobile (10) se soulève de la surface de base (1),
**caractérisé en ce que**
le composant mobile (10) présente au moins une languette (10) avec une extrémité proximale (11), une section intermédiaire (12) et une extrémité distale (13), dans lequel la au moins une languette (10) est maintenue de façon à pouvoir coulisser dans la zone de son extrémité distale (13) au niveau de la surface de base (1) et
au moins une section coudée (14, 15, 16) est prévue, fixée par son extrémité distale sur la section intermédiaire (12) de la au moins une languette (10) et fixée par son extrémité proximale sur la surface de base (1),
dans lequel, au niveau de la au moins une section coudée (14, 15, 16), se trouve un support d'aiguille (2) pour une aiguille chirurgicale et
dans lequel, en tirant sur la languette (10), il se produit un soulèvement du support d'aiguille (2) depuis la surface de base (1).

2. Lève-aiguille pour matériel de suture chirurgicale selon la revendication 1,
dans lequel la au moins une section coudée (14, 15, 16) dispose d'au moins un point de courbure théorique (17, 18, 19), qui s'étend de manière essentiellement transversale à l'axe longitudinal de la au moins une languette (10).

3. Lève-aiguille pour matériel de suture chirurgicale selon la revendication 1 ou 2, dans lequel
la au moins une section coudée (14, 15, 16) dispose de trois points de courbure théorique (17, 18, 19) et le support d'aiguille (2) se compose d'un support à trois points, qui place l'aiguille de telle sorte qu'elle traverse le point de courbure théorique (18) médian.

4. Lève-aiguille pour matériel de suture chirurgicale comprenant :
une surface de base (1) et
un composant mobile (10), qui est mobile au moins partiellement par rapport à la surface de base (1) dans le plan de la surface de base (1),
dans lequel le composant mobile (10) est formé et fixé à la surface de base (1) de telle sorte qu'un mouvement du composant mobile (10) dans le plan de la surface de base (1) entraîne qu'au moins une zone (14, 15) du composant mobile (10) se soulève de la surface de base (1),
**caractérisé en ce que**
le composant mobile (10) présente au moins une languette (10) avec une extrémité proximale (11), une section intermédiaire (12) et une extrémité distale (13), dans lequel la au moins une languette (10) est fixée à la surface de base (1) dans la zone de son extrémité distale (13) et la au moins une languette (10) est maintenue de manière à pouvoir coulisser sur la surface de base (1) dans la zone de son extrémité proximale (11),
dans lequel la section intermédiaire (12) est formée comme au moins une section en forme de "S", qui relie l'extrémité proximale (11) et l'extrémité distale (13) de la languette (10),
dans lequel, au niveau de la au moins une section en forme de "S", un support d'aiguille (2) est prévu, pour une aiguille chirurgicale et
dans lequel le fait de tirer sur la languette (10) entraîne que le support d'aiguille (2) se soulève de la surface de base (1).

5. Lève-aiguille pour matériel de suture chirurgicale selon la revendication 4,
dans lequel le support d'aiguille (2) est composé d'un support à trois points, qui dispose l'aiguille de telle sorte que l'extrémité de l'aiguille opposée à la pointe de l'aiguille s'étend en dehors de la zone en forme de "S" jusqu'à l'extrémité proximale (11) de la au moins une languette (10).

6. Lève-aiguille pour matériel de suture chirurgicale selon l'une des revendications 1 à 5,
dans lequel la au moins une languette (10) dispose d'un élément de poignée (20).

7. Lève-aiguille pour matériel de suture chirurgicale selon une des revendications 1 à 6,
dans lequel la au moins une languette (10) et/ou la surface de base (1) disposent d'un agencement (21, 22, 23), qui maintient la au moins une languette (10), après un transfert de celle-ci, dans la position transférée.

8. Lève-aiguille pour matériel de suture chirurgicale selon une des revendications 1 à 7,
dans lequel la au moins une languette (10) est maintenue de manière à pouvoir coulisser sur la surface de base (1), en ce qu'un composant de pont (24) s'étend sur la au moins une languette (10), lequel composant est fixé des deux côtés de la au moins une languette (10) sur la surface de base (1), dans lequel le composant de pont (24) est lui-même de préférence une pièce de la au moins une languette (10), qui est non pliée, non courbée ou non coudée, est plus large que la zone limite de la au moins une languette (10) et repose sur une pièce de la au moins une languette (10).

9. Lève-aiguille pour matériel de suture chirurgicale selon une des revendications 1 à 8,
dans lequel la au moins une languette (10) est maintenue de manière à pouvoir coulisser sur la surface de base (1), en ce que des rabats en porte-à-faux (25) sont formés dans la surface de base (1), lesquels sont agencés des deux côtés de la au moins une languette (10), dans lequel la au moins une languette (10) est agencée entre les extrémités libres des rabats en porte-à-faux (25) et de la surface de base (1).

10. Lève-aiguille pour matériel de suture chirurgicale selon une des revendications 1 à 9,
dans lequel la au moins une languette (10) est maintenue de manière à pouvoir coulisser sur la surface de base (1), en ce qu'une fente (26) est prévue dans la au moins une languette (10) le long de son axe longitudinal et que des rabats en porte-à-faux (25) sont formés dans la surface de base (1), qui s'étendent dans les deux directions de manière transversale à l'axe longitudinal de la au moins une languette (10) à travers la fente (26) et dans lequel la au moins une languette (10) est agencée entre les extrémités libres des rabats en porte-à-faux (25) et de la surface de base (1).

11. Emballage de matériel de suture comportant :
un composant fond et
un composant couvercle, dans lequel un espace de réception est formé au moins partiellement pour les fils entre le composant fond et le composant couvercle,
une surface de base (1), qui est prévue sur le composant fond ou le composant couvercle et
un lève-aiguille selon l'une des revendications précédentes.

12. Emballage de matériel de suture selon la revendication 11, dans lequel
la surface de base (1) est prévue sur le composant fond et le lève-aiguille se trouve entre le composant fond et le composant couvercle, de sorte que les zones de la languette (10), qui sont maintenues de manière à pouvoir coulisser contre la plaque de base (1), sont maintenues par le biais du composant couvercle.

13. Emballage de matériel de suture selon la revendication 11 ou 12, dans lequel
un composant étiquette est prévu sur le composant couvercle, qui recouvre au moins la pointe de l'aiguille même lorsque le lève-aiguille est actionné.
